# EUROPEAN PATENT SPECIFICATION

(11) **EP 1 423 047 B1**
(45) Date of publication and mention of the grant of the patent: **27.06.2007**
(21) Application number: 02755282.7
(22) Date of filing: 30.08.2002
(51) Int. Cl.: A61B 5/03, A61M 25/00

(54) **MULTI-LUMEN, MANOMETRY CATHETERS**
KATHETER ZUR DRUCKMESSUNG MIT MEHREREN LEITUNGEN
CATHETERS MANOMETRIQUES MULTILUMIERE

(30) Priority: 06.09.2001 GB 0121544
(43) Date of publication of application: 02.06.2004
(73) Proprietor: Mediplus Limited, High Wycombe, Buckinghamshire, HP12 3SU (GB)
(72) Inventor: URIE, Robert, Graham, Nr. Amersham, Buckinghamshire HP7 0QQ (GB)
(74) Representative: Charig, Raymond Julian
(86) International application number: PCT/GB2002/003980
(87) International publication number: WO 2003/022343

(56) References cited:
- US-A- 5 178 803
- US-A- 5 433 216
- US-A- 5 609 060
- US-A- 5 830 196

## Description

The present invention relates to mult-lumen tubes for use as manometry catheters, and in particular to improvements in the connection mechanisms for such catheters.

Various medical diagnosis procedures require the use of manometers that are capable of measuring pressure simultaneously at multiple locations along the length of a tube. For example, in gastrointestinal manometry, a manometry catheter consists of a multi-lumen tube in which each individual lumen communicates with a lumen opening, with the multiple lumen openings being located at various points along the length of the tube.

The multi-lumen tube, with its plurality of lumen openings, is positioned within the tube cavity in which pressure is to be measured, for example, the oesophagus. The lumen openings enable sensing of the pressure in various parts of the oesophagus along its length. As peristaltic waves from the oesophagus apply pressure to varying positions along the tube, these pressures are communicated to pressure transducers coupled to a distal end of each lumen.

In a tonometric multi-lumen catheter, as described in US 5,433,216, the catheter includes one or more balloon-like chambers enclosing respective openings in the wall of the catheter tube. In use, each chamber is filled with a pressure transmitting medium which causes the chamber to expand to contact the wall of an organ under examination. The medium is in pressure transmitting communication with a respective lumen in the catheter tubing by virtue of the corresponding opening, and fills the entire length of the respective lumen and associated tubing which emerges from the end of the catheter. In this way, a 'closed pressure system' is formed in each lumen, having a single uninterrupted volume of pressure transmitting medium, which may be monitored by a pressure sensing means and/or by optional sensors inside the chamber. The end of the catheter at which the tubing emerges is typically wider than the body of the catheter.

To obtain comprehensive and useful pressure readings from many positions along the oesophagus, a corresponding number of lumens must be used within the multi-lumen tube. In a typical example, a nine-lumen tube is used.

A problem encountered is that the multi-lumen tube to be inserted into the patient's body must of necessity be sufficiently small in diameter so that patient discomfort is minimised, so that undue distortion of the oesophagus is avoided and so that adverse effects on the pressure measurement are also minimised.

Although small diameter multi-lumen tubes can readily be formed from suitable flexible plastics materials, easy and reliable connection of each of the many lumens, at a distal end of the catheter, to separate pressure transducers can be a problem owing to the very small internal diameter of the individual lumens.

The present invention aims to provide an improved means for coupling the individual lumens of a multi-lumen catheter tube at a distal end thereof to individual pressure transducer couplings.

According to one aspect, the present invention provides a manometry catheter according to claim 1.

Embodiments of the present invention will now be described by way of example and with reference to the accompanying drawings in which:
Figure 1a is a cross-sectional end view of a nine-lumen manometry catheter of the prior art;
Figure 1b is a schematic side cross-sectional view of a manometry catheter apparatus including connecting device according to the prior art, with the catheter shown in cross section on A-A;
Figure 1c is a schematic close up side cross-sectional view of the connecting device of Figure 1b;
Figure 2a is a schematic side view of a manometry catheter apparatus according to one embodiment of the present invention;
Figure 2b is a cross-sectional end view of the distal end of the catheter in Figure 2a; and
Figure 2c is a cross-sectional end view of the proximal end of the catheter of Figure 2a.

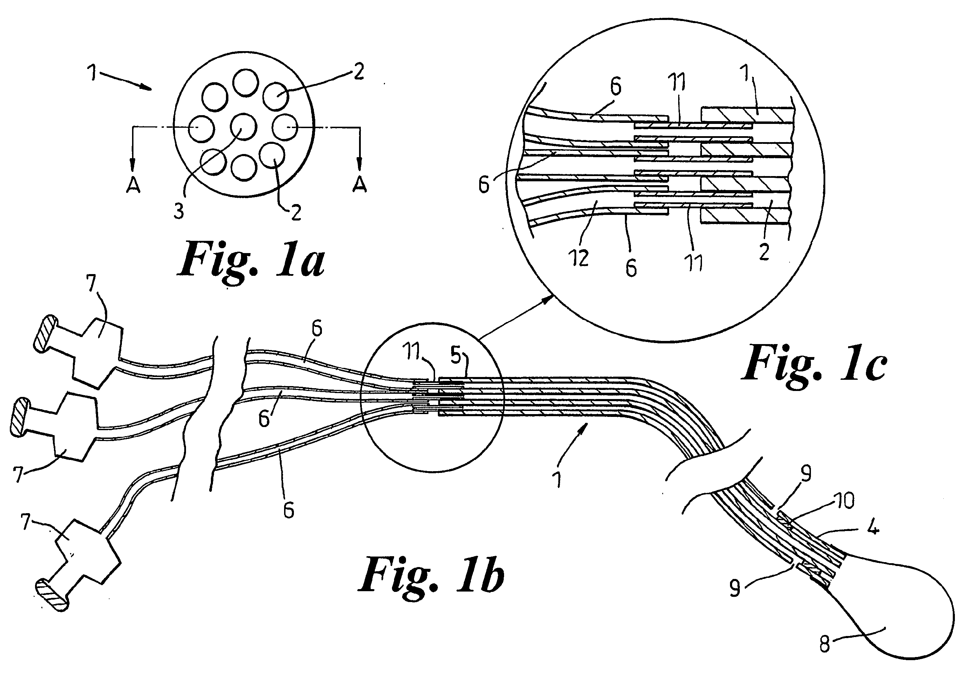

With reference to Figure 1a, a conventional multi-lumen tube catheter 1 has a diameter of approximately of 5 mm. The catheter has eight peripheral lumens 2 and one central lumen 3 each having an internal diameter of approximately 1 mm. The catheter is conventionally extruded in a flexible material such as PVC, using known extrusion processes.

With reference to Figure 1b, a conventional manometry apparatus includes a multi-lumen catheter 1 extending from a proximal end 4 to a distal end 5. The apparatus further includes a plurality of flexible connection tubes 6 each connected at a first end to the distal end 5 of the multi-lumen catheter 1 and coupled at the second end to a plug 7. Each plug 7 is adapted to be received by, and form an airtight seal with, a pressure sensing device.

At the proximal end 4 of the catheter 1 an inflation device, such as balloon 8 is coupled to the multi-lumen catheter 1 by way of a friction fit and/or adhesive or other suitable means. The balloon may be inflated by way of the central lumen 3, coupled to one of the flexible connection tubes 6 and corresponding plug 7.

To fulfil the objective of simultaneous pressure measurement at multiple positions along the length of the catheter 1, each peripheral lumen 2 communicates with a lumen opening 9 comprising an aperture in the outer wall of the multi-lumen tube 1. Adjacent to the lumen opening 9, and between the lumen opening and the proximal end of the catheter 1 the remaining portion of the lumen 2 is occluded by an occlusion 10 to prevent communication with the proximal end of the catheter 1. The occlusion 10 may be any suitable device or substance adequate to form an airtight seal within the lumen, such as a pin inserted into the proximal end 4 and glued into place.

With reference to Figure 1c, to couple the catheter 1 to the flexible connection tubes 6 a plurality of hollow cylindrical pins 11 are engaged in each lumen 2, 3 of the multi-lumen catheter 1, and correspondingly into the lumen 12 of a respective flexible connection tube 6. Typically, the hollow cylindrical pins 11 are formed of stainless steel and are glued into place.

It will be noted that the connection of nine stainless steel pins 11 in between the multi-lumen catheter 1 and the plurality of flexible connection tubes 6 incurs manufacturing costs and assembly costs in producing such fine, high quality metal components and installing them accordingly.

It will also be noted that the hollow cylindrical pins 11 also impose a reduced internal diameter on the lumen communication between the catheter 1 and the flexible connection tubes 6. Of necessity, the internal diameter of the hollow cylindrical pins 11 must be less than that of either the catheter lumens 2, 3 or the lumen 12 of the flexible connection tubes 6. This is disadvantageous for a number of reasons.

Firstly, it effectively imposes a lower limit on the internal diameter of the lumens being connected, according to the availability of small diameter hollow cylindrical pins. This limits the number of lumens that can be provided within a catheter of an external diameter dictated by the medical conditions in which it is to be used.

Secondly, the reduced internal diameter of the hollow cylindrical pins has adverse effects on the fluid flow through the combined lumen / connector system. If insufficient fluid flow is achieved through the limited internal diameter of the hollow cylindrical pins, this has an adverse effect on the accuracy and resolution of pressure measurements taken therethrough. Similarly, if the internal diameter of the hollow cylindrical pins becomes too small, capillary action can start to have adverse effects on the fluid flows upon which pressure measurements are based, resulting in inaccuracies in the measurements.

It will be shown that the present invention avoids the use of the hollow cylindrical pins 11 to connect the multi-lumen catheter 1 to the flexible connection tubes 6.

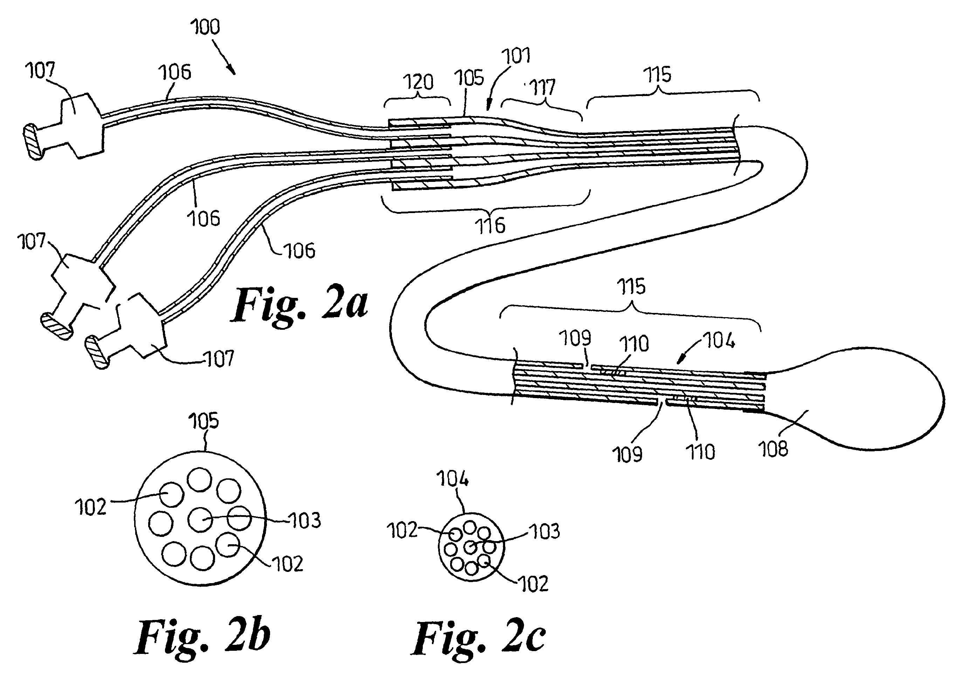

With reference to Figure 2a, a manometry apparatus 100 according to a presently preferred embodiment of the invention includes a multi-lumen catheter 101 which has a proximal end 104 and a distal end 105 respectively seen in cross-section in figures 2c and 2b. The proximal end 104 comprises a proximal portion 115 that has a first diameter. The distal end 105 comprises a distal portion 116 that has a second diameter that is larger than the first diameter.

Preferably, the proximal portion 115 extends over most of the length of the catheter, eg. all excepting about 2 to 7.5 cm, or more preferably, about 2 to 5 cm at the distal end 105 Preferably, the distal portion 116 extends over a short length of the catheter, eg. between 1 or 2 and 10 cm, or more preferably between 1 and 5 cm, or between 2 and 5 cm. Preferably, the external diameter of the proximal portion 115 is approximately 5 mm and the external diameter of the distal portion 116 is approximately 8 mm.

In the preferred embodiment, the catheter 101 has eight peripheral lumens 102 and one central lumen 103. Preferably, the peripheral lumens 102 each have an internal diameter in the proximal portion 115 of between 0.4 mm and 1 mm, and an internal diameter in the distal portion of approximately 2 mm. In one arrangement, the central lumen 103 is conveniently slightly larger, eg. between 0.7 mm and 1.5 mm internal diameter in the proximal portion 104 and approximately 2.5 mm internal diameter in the distal portion 105.

Currently preferred sizes (diameters) of nine-lumen catheter required for various medical applications are as shown in the table below:

| TYPE | OUTSIDE Ø | | LUMEN INTERNAL Ø | | | |
|---|---|---|---|---|---|---|
| | | | PROXIMAL END | | DISTAL END | |
| | PROXIMAL END | DISTAL END | CENTRAL | PERIPHERAL | CENTRAL | PERIPHERAL |
| A | 4.9 mm | 7.9 mm | 1.5 mm | 0.85 mm | 2.5 mm | 2.0 mm |
| B | 3.9 mm | 7.9 mm | 1.2 mm | 0.6 mm | 2.5 mm | 2.0 mm |
| C | 2.3 mm | 7.9 mm | 0.74 mm | 0.46 mm | 2.5 mm | 2.0 mm |

The catheter is preferably extruded in a flexible material such as PVC, polyurethane or any suitable medical grade thermoplastic polymer according to known extrusion processes.

In the present invention, it has been recognised that conventional extrusion process techniques can cheaply and easily be deployed to increase the diameter of the catheter 101 by slowing up the extrusion process for the final few centimetres of the catheter, resulting in a tapering outward, then final larger, diameter of catheter. It is also possible, during the extrusion process, to apply an increased air pressure in the lumens of the tube being extruded, to control the increase in diameter of the catheter and its lumens.

In general, both the lateral separation of the individual lumens and the internal diameter of the individual lumens increase between the proximal portion 115 and the distal portion 116. Preferably, this increase occurs in a taper zone 117 resulting from a gradual change in the extrusion process.

The manometry apparatus 100 includes the multi-lumen catheter 101 together with a plurality of flexible connection tubes 106 each connected at a first end to the distal end 105 of the multi-lumen catheter 101 and coupled at the second end to a plug 107. Each plug 107 is adapted to be received by, and form an airtight seal with, a pressure sensing device (not shown).

For certain medical applications, such as use of catheters for ano-rectal measurements, an inflation device, such as balloon 108 may be coupled to the proximal end 104 of the multi-lumen catheter 101 by way of a friction fit and/or adhesive or other suitable means. The balloon may be inflated by way of the central lumen 103, coupled to one of the flexible connection tubes 106 and corresponding plug 107.

Each peripheral lumen 102 communicates with a lumen opening 109 comprising an aperture in the outer wall of the multi-lumen tube 101. Adjacent to each lumen opening 109, and between the lumen opening and the proximal end of the catheter 101, the remaining portion of the lumen 102 is occluded by an occlusion 110 to prevent communication with the proximal end of the catheter. The occlusion 110 may be any suitable device or substance adequate to form an airtight seal within the lumen, such as a pin inserted into the proximal end 104 and glued into place. Alternatively, the occlusion 110 may be formed by injected glue or resin.

The invention provides for simpler and more efficient coupling of the multi-lumen catheter 101 to the flexible connection tubes 106. Because the distal portion 116 of the multi-lumen catheter is expanded during the extrusion process, direct coupling of each of the flexible connection tubes 106 into a corresponding lumen 102, 103 of the multi-lumen catheter 101 is possible. A simple friction fit between the overlapping lumen 102, 103 walls and tube 106 walls in a connection zone 120 may be used, or more preferably, an adhesive may be used.

It will be noted that the direct insertion of flexible connection tubes 106 into the lumens of the multi-lumen catheter 101 ensures that the smallest lumen diameter encountered between the lumen openings 109 and the pressure sensor plugs 107 is the internal diameter of the flexible connection tubes 106. There is no intermediate restriction of any connecting pins 11.

It will also be noted that the manufacturing costs of fine, high quality metal components 11 and the associated assembly costs for installing them are accordingly avoided.

Although the invention has been described and illustrated in the context of a nine-lumen manometry apparatus, it will be understood that the principle applies to other numbers of lumens in a multi-lumen catheter.

Other embodiments are within the scope of the appended claims.

## Claims

1. A manometry catheter (101) comprising:
an extruded flexible tube having a plurality of lumens (102, 103) extending through the body of the tube between a proximal end (104) and a distal end (105), the extruded flexible tube having a first diameter in a proximal portion (115) extending over a part of its length at the proximal end and a second diameter, larger than the first diameter, in a distal portion (116) extending over a part of its length at the distal end (105), and
a plurality of flexible connection tubes (106) each being coupled to a respective one of the plurality of lumens (102, 103) by insertion of the respective flexible connection tube (106) into a corresponding lumen (102,103) at the distal end (105) of the catheter,
**characterised in that**
wherein the internal diameter of the individual lumens (102, 103) within the extruded flexible tube is increased within the distal portion (116) while maintaining substantially the same cross-sectional shape.

2. The manometry catheter of claim 1 in which the proximal portion (115) extends for a substantial part of the length of the catheter (101) and the distal portion (116) extends for a relatively small part of the length of the catheter (101).

3. The manometry catheter of claim 2 in which the distal portion (116) has a length in the range 1 to 10 cm.

4. The manometry catheter of claim 2 in which the distal portion (116) has a length in the range 1 to 5 cm.

5. The manometry catheter of any preceding claim in which the lateral separation of the individual lumens (102, 103) within the flexible tube is increased within the distal portion.

6. The manometry catheter of any preceding claim in which the outside diameter of the proximal portion (115) lies in the range approximately 2mm to 5 mm and the outside diameter of the distal portion (116) is approximately 8 mm.

7. The manometry catheter of any preceding claim in which the inside diameter of lumens (102, 103) located adjacent the periphery of the catheter (101) lies in the range approximately 0.74 mm to 1.5 mm in the proximal portion (115) and approximately 2 mm in the distal portion (116).

8. The manometry catheter of any preceding claim further including a lumen opening (109) communicating with each of a plurality of said lumens (102, 103) extending through the catheter walls to an outer surface thereof.

9. The manometry catheter of claim 8 in which each lumen opening (109) is located at a different longitudinal position along the catheter (101).

10. The manometry catheter of claim 1 in which the distal portion (116) includes a tapered region (117) between the distal end (105) and the proximal portion (115).

## Patentansprüche

1. Manometriekatheter (101), umfassend:
einen extrudierten biegsamen Schlauch mit mehreren Lumina (102, 103), die durch den Körper des Schlauchs zwischen einem proximalen Ende (104) und einem distalen Ende (105) verlaufen, wobei der extrudierte biegsame Schlauch einen ersten Durchmesser in einem proximalen Abschnitt (115), der sich über einen Teil seiner Länge an dem proximalen Ende erstreckt, und einen zweiten Durchmesser, der größer als der erste Durchmesser ist, in einem distalen Abschnitt (116) aufweist, der sich über einen Teil seiner Länge an dem distalen Ende (105) erstreckt, und
mehrere biegsame Verbindungsschläuche (106), von denen jeder mit einem jeweiligen der mehreren Lumina (102, 103) durch Einsetzen des jeweiligen biegsamen Verbindungsschlauchs (106) in ein entsprechendes Lumen (102, 103) an dem distalen Ende (105) des Katheters verbunden ist,
**dadurch gekennzeichnet, dass** der Innendurchmesser der einzelnen Lumina (102, 103) innerhalb des extrudierten biegsamen Schlauchs in dem distalen Abschnitt (116) erhöht ist, während im Wesentlichen die gleiche Querschnittsform beibehalten wird.

2. Manometriekatheter nach Anspruch 1, wobei der proximale Abschnitt (115) über einen wesentlichen Teil der Länge des Katheters (101) verläuft und der distale Abschnitt (116) über einen relativ kleinen Teil der Länge des Katheters (101) verläuft.

3. Manometriekatheter nach Anspruch 2, wobei der distale Abschnitt (116) eine Länge im Bereich von 1 bis 10 cm aufweist.

4. Manometriekatheter nach Anspruch 2, wobei der distale Abschnitt (116) eine Länge im Bereich von 1 bis 5 cm aufweist.

5. Manometriekatheter nach einem der vorhergehenden Ansprüche, wobei die seitliche Trennung der einzelnen Lumina (102, 103) innerhalb des biegsamen Schlauchs in dem distalen Abschnitt erhöht ist.

6. Manometriekatheter nach einem der vorhergehenden Ansprüche, wobei der Außendurchmesser des proximalen Abschnitts (115) im Bereich von etwa 2 mm bis 5 mm liegt und der Außendurchmesser des distalen Abschnitts (116) etwa 8 mm beträgt.

7. Manometriekatheter nach einem der vorhergehenden Ansprüche, wobei der Innendurchmesser der Lumina (102, 103), die benachbart zu der Peripherie des Katheters (101) angeordnet sind, im Bereich von etwa 0,74 mm bis 1,5 mm in dem proximalen Abschnitt (115) liegt und etwa 2 mm in dem distalen Abschnitt (116) beträgt.

8. Manometriekatheter nach einem der vorhergehenden Ansprüche, ferner mit einer Lumenöffnung (109), die mit jedem der mehreren Lumina (102, 103) in Verbindung steht und durch die Katheterwände zu einer äußeren Oberfläche davon verläuft.

9. Manometriekatheter nach Anspruch 8, wobei jede Lumenöffnung (109) an einer anderen Längsposition entlang des Katheters (101) angeordnet ist.

10. Manometriekatheter nach Anspruch 1, wobei der distale Abschnitt (116) einen verjüngten Bereich (117) zwischen dem distalen Ende (105) und dem proximalen Ende (115) aufweist.

## Revendications

1. Cathéter manométrique (101) comprenant :
un tube flexible extrudé ayant une pluralité de lumens (102,103) s'étendant à travers le corps du tube entre une extrémité proximale (104) et une extrémité distale (105), le tube flexible extrudé ayant un premier diamètre dans une portion proximale (115) s'étendant sur une partie de sa longueur à l'extrémité proximale et un second diamètre, plus grand que le premier diamètre, dans une portion distale (116) s'étendant sur une partie de sa longueur à l'extrémité distale (105), et
une pluralité de tubes de connexion flexibles (106) chacun étant couplé à une des pluralités de lumens (102,103) respective par insertion du tube de connexion flexible respectif (106) dans un lumen correspondant (102,103) à l'extrémité distale (105) du cathéter, **caractérisé en ce que** le diamètre interne des lumens individuels (102,103) à l'intérieur du tube flexible extrudé est augmenté dans la portion distale (116) tout en conservant substantiellement la même forme en coupe transversale.

2. Cathéter manométrique selon la revendication 1, dans lequel la portion proximale (115) s'étend sur une partie substantielle de la longueur du cathéter (101) et la portion distale (116) s'étend sur une partie relativement faible de la longueur du cathéter (101).

3. Cathéter manométrique selon la revendication 2, dans lequel la portion distale (116) a une longueur dans le domaine de 1 à 10 cm.

4. Cathéter manométrique selon la revendication 2, dans lequel la portion distale (116) a une longueur dans le domaine de 1 à 5 cm.

5. Cathéter manométrique selon l'une des revendications précédentes, dans lequel la séparation latérale des lumens individuels (102,103) dans le tube flexible est augmentée dans la portion distale.

6. Cathéter manométrique selon l'une des revendications précédentes, dans lequel le diamètre extérieur de la portion proximale (115) se trouve dans le domaine d'approximativement 2mm à 5 mm et le diamètre extérieur de la portion distale (116) est d'approximativement 8 mm.

7. Cathéter manométrique selon l'une des revendications précédentes, dans lequel le diamètre interne des lumens (102,103) situés de façon adjacente à la périphérie du cathéter (101) se trouve dans le domaine approximativement de 0,74 mm à 1,5 mm dans la portion proximale (115) et approximativement 2 mm dans la portion distale (116).

8. Cathéter manométrique selon l'une des revendications précédentes, incluant en outre une ouverture de lumen (109) communiquant avec chacune d'une pluralité desdits lumens (102,103) s'étendant à travers les parois du cathéter vers une surface externe de celui-ci.

9. Cathéter manométrique selon la revendication 8, dans lequel chaque ouverture de lumen (109) est située à une position longitudinale différente le long du cathéter (101).

10. Cathéter manométrique selon la revendication 1, dans lequel la portion distale (116) inclut une région conique (117) entre l'extrémité distale (105) et la portion proximale (115).
